(19) Europäisches Patentamt
European Patent Office
Office européen des brevets

(11) Numéro de publication: **0 237 516**
**A1**

# (12) DEMANDE DE BREVET EUROPEEN

(21) Numéro de dépôt: **87870032.7**

(22) Date de dépôt: **09.03.87**

(51) Int. Cl.[4]: **C 07 D 239/49**
A 61 K 31/505

(30) Priorité: **13.03.86 FR 8603602**

(43) Date de publication de la demande:
**16.09.87 Bulletin 87/38**

(84) Etats contractants désignés:
**AT BE CH DE ES FR GB GR IT LI LU NL SE**

(71) Demandeur: **COMPAGNIE ROUSSELOT, Société Anonyme:**
**8, rue Christophe Colomb**
**F-75008 Paris (FR)**

(72) Inventeur: **Giral, Louis**
**Les 2 Ruisseaux No. 2 1526 Avenue du Père Soulas**
**F-34000 Montpellier (FR)**

**Calas, Michèle**
**Rue de la Traversière 9**
**F-34980 Saint-Gely-du-Fesc (FR)**

**Puygrenier, Marc**
**Rue de Vendargues 17**
**F-34170 Clapiers (FR)**

(74) Mandataire: **Cauchie, Daniel**
**c/o S.A. LABAZ-SANOFI N.V. Avenue De Béjar, 1**
**B-1120 Bruxelles (BE)**

## (54) Dérivés de benzyl-pyrimidine, leur procédé de préparation ainsi que les compositions en contenant.

(57) L'invention se rapporte à de nouveaux dérivés de benzyl-pyrimidine de formule générale :

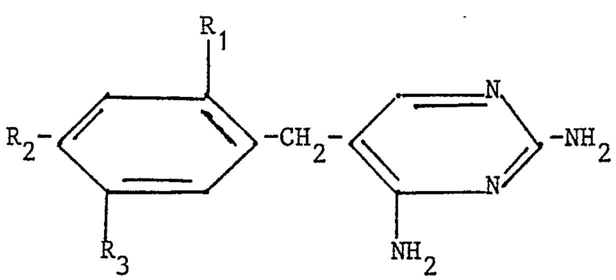

ainsi qu'à leurs sels biologiquement acceptables, dans laquelle $R_1$ représente un atome de brome ou d'iode, $R_2$ représente un groupement isopropyle, éthoxy ou cycloalkoxy ayant de 3 à 6 atomes de carbone et $R_3$ représente un radical alkoxy ayant de 1 à 3 atomes de carbone à condition que lorsque $R_2$ représente éthoxy et $R_3$ représente méthoxy, $R_1$ représente iode.

Les dérivés de l'invention se sont révélés utiles comme agents antibactériens, antiparasitaires et antipaludéens.

EP 0 237 516 A1

**Description**

DERIVES DE BENZYL-PYRIMIDINE, LEUR PROCEDE DE PREPARATION AINSI QUE LES COMPOSITIONS EN CONTENANT

L'invention se rapporte, d'une manière générale, à de nouveaux dérivés de benzyl-pyrimidine, à leur procédé de préparation ainsi qu'aux compositions en contenant.

En particulier, l'invention se rapporte aux dérivés de benzyl-pyrimidine de formule générale :

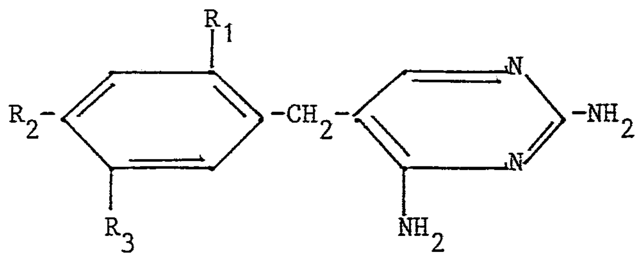

I

dans laquelle $R_1$ représente un atome de brome ou d'iode, $R_2$ représente un groupement isopropyle, éthoxy ou cycloalkoxy ayant de 3 à 6 atomes de carbone, par exemple cyclopentyloxy ou cyclohexyloxy et $R_3$ représente un radical alkoxy ayant de 1 à 3 atomes de carbone par exemple méthoxy à condition que lorsque $R_2$ représente éthoxy et $R_3$ représente méthoxy, $R_1$ représente iode.

L'invention concerne également les sels biologiquement acceptables des dérivés de benzyl-pyrimidine de formule I formés à partir d'acides inorganiques comme les acides sulfurique, phosphorique ou chlorhydrique ou à partir d'acides organiques tels que les acides acétique, tartrique ou citrique.

Par "sels biologiquement acceptables" on entend également des sels hydrosolubles des dérivés de benzyl-pyrimidine de formule I tels que les sels obtenus à partir d'un acide carboxylique ou sulfonique contenant un ou plusieurs groupements hydroxyles tels que l'acide gluconique, lactobionique, pantothénique ou iséthionique.

Les dérivés de benzyl-pyrimidine de l'invention se sont révélés doués de propriétés pharmacologiques particulièrement intéressantes en particulier des propriétés antibactériennes spécifiques des bactéries Gram +, antiparasitaires et antipaludéennes.

en outre, en association à des dérivés de sulfamide tels que sulfadiazine, sulfadiméthoxine, sulfaméthoxazole, sulfamoxol, sulfamidine ou sulfaméthoxypyridazine, les dérivés de benzyl-pyrimidine de l'invention présentent une synergie importante.

En conséquence, un autre objet de l'invention se rapporte à des compositions pharmaceutiques ou vétérinaires contenant comme principe actif au moins un dérivé de benzyl-pyrimidine de l'invention, associé ou non à un second principe actif tel qu'un dérivé de sulfamide, ainsi qu'un véhicule pharmaceutique ou un excipient approprié.

Parmi les composés de formule I, ceux dans lesquels $R_2$ représente un groupement cycloalkoxy constituent une classe préférée de composés.

Les dérivés de benzyl-pyrimidine de l'invention peuvent être préparés par halogénation en présence d'acide acétique, d'un dérivé de benzyl-pyrimidine de formule générale :

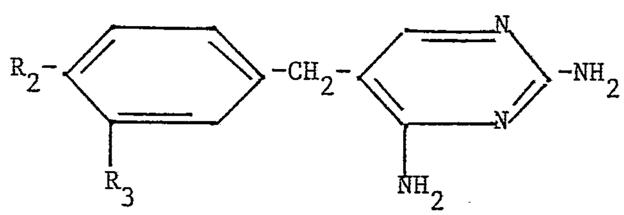

II

dans laquelle $R_2$ et $R_3$ ont la même signification que dans la formule I, avec le brome ou le monochlorure d'iode pour obtenir le composé désiré sous forme basique que l'on fait réagir si on le désire avec un acide organique ou inorganique approprié pour obtenir un sel biologiquement acceptable.

L'halogénation au moyen de brome a lieu à température ambiante tandis que celle avec le monochlorure d'iode s'effectue en milieu acide acétique aqueux à température d'ébullition du milieu.

Les composés de formule II sont soit des composés connus ayant été décrits dans Eur. J. Med. Chem. - Chim. Ther. 1982, 17, No. 6 pp. 497-504 soit des composés pouvant être préparés par la méthode qui y est décrite à savoir par condensation en milieu basique d'un aldéhyde de formule générale :

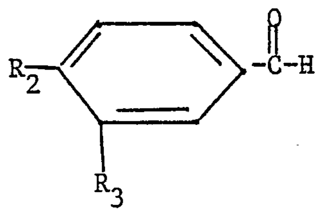

III

dans laquelle $R_2$ et $R_3$ ont la même signification que précédemment, avec le β-anilinopropionitrile, le dérivé β-anilino α-benzyl-acrylonitrile formé étant par la suite cyclisé avec un excès de chlorhydrate de guanidine en milieu basique.

La diamino-2,4 (triméthoxy-3,4,5 benzyl)-5 pyrimidine plus connue sous le nom de triméthoprime constitue un agent antibactérien à spectre large qui, associé à certains autres antibactériens tels que des dérivés de sulfamide, présente un effet synergique important.

Cependant, comme la plupart des composés antibactériens, la triméthoprime peut provoquer l'apparition de résistances vis-à-vis de certains germes pathogènes initialement détruits par ce composé.

En outre, on a décrit dans Eur. J. Med. Chem. - Chim. Ther. 1982, 17, No. 6 pp. 497-504, une série de dérivés de benzyl-pyrimidine, analogues de triméthoprime ainsi que leurs propriétés antibactériennes à l'égard de germes pathogènes Gram + et Gram -.

Alors que la référence en question ne fait aucune allusion au comportement de ces dérivés en présence de souches Gram + résistant à la triméthoprime, on a testé, dans le cadre de la présente invention, les dérivés de benzyl-pyrimidine en question vis-à-vis de germes de ce type.

Aucune activité antibactérienne appréciable n'a cependant été enregistrée vis-à-vis de telles souches résistantes notamment vis-à-vis de souches résistantes de Staphylococcus aureus.

On a maintenant trouvé, de manière surprenante, que les dérivés de benzyl-pyrimidine de l'invention se sont révélés capables d'activité antibactérienne importante à l'égard de souches pathogènes Gram + résistant à la triméthoprime tout en étant également plus actives que ce composé à l'égard d'un nombre élevé de souches pathogènes sensibles à la triméthoprime.

Par conséquent, seuls ou en association, les composés de l'invention pourront être utiles dans des cas où la triméthoprime fait défaut. En outre, vis-à-vis de souches pathogènes sensibles, ces mêmes composés de l'invention pourront être administrés à des doses plus faibles que celles préconisées pour la triméthoprime.

Dans des associations synergiques de triméthoprime et de dérivé de sulfamide, les réactions secondaires enregistrées sont dues le plus souvent au sulfamide présent. C'est pourquoi, il apparaît intéressant de pouvoir disposer de composés plus actifs que la triméthoprime de manière à pouvoir diminuer la quantité de sulfamide sans nuire à l'efficacité de l'association.

Les dérivés de benzyl-pyrimidine de l'invention répondent à cet objectif étant effectivement plus actifs que la triméthoprime à l'égard de bactéries Gram +.

Enfin, la toxicité présentée par les composés de l'invention s'est montrée du même ordre que celle de la triméthoprime et en tout état de cause compatible avec leur utilisation à des fins thérapeutiques.

A cet effet, les composés de l'invention peuvent être administrés à la dose journalière de 2 mg à 5 mg/kg dans les infections bactériennes spécifiques des bactéries à Gramt et notamment dans le traitement des infections staphylococciques résistant à la triméthoprime.

De ce point de vue, les dérivés de benzyl-pyrimidine de l'invention qui se sont révélés les plus intéressants sont :

la diamino-2,4 (iodo-2 éthoxy-4 méthoxy-5 benzyl)-5 pyrimidine (Ex. 1)
la diamino-2,4 (bromo-2 cyclopentyloxy-4 méthoxy-5 benzyl)-5 pyrimidine (Ex. 7)
la diamino-2,4 (iodo-2 cyclopentyloxy-4 méthoxy-5 benzyl)-5 pyrimidine (Ex. 4)
la diamino-2,4 (bromo-2 cyclohexyloxy-4 méthoxy-5 benzyl)-5 pyrimidine (Ex. 22)
ainsi que leurs sels pharmaceutiquement acceptables tels que l'iséthionate ou le lactobionate.

Des tests effectués in vitro suivant les protocoles expérimentaux ci-dessous ont permis de mettre en évidence l'activité antibactérienne des dérivés de l'invention.

A cet effet, on a déterminé l'activité bactériostatique par la méthode des antibiogrammes par dilution en milieu gélosé (milieu de MUELLER-HINTON déshydraté) et par diffusion en milieu gélosé.

On a utilisé le composé à étudier selon une gamme de concentrations de 0,625 à 640 µg/ml (progression de raison 2) à partir de solutions-mères titrant 640 µg/ml à 1280 µg/ml selon la solubilité du composé, toutes les dilutions ultérieures ayant été réalisées dans un mélange méthanol/eau à 20%.

I.Détermination des concentrations minimales inhibitrices (CMI)

On a procédé sur milieu gélosé de MUELLER-HINTON déshydraté, par la méthode des dilutions sériées, les concentrations finales en composé à étudier s'échelonnant de 0,0625 à 32 ou 64 µg/ml.

A cet effet, on ensemence des boîtes de PETRI à l'aide d'un inoculateur multiple (inoculum de $5 \times 10^3$ à $1 \times 10^4$ unités formant colonies par spot d'inoculation) et on enregistre le résultat après 18 h à 37° C en ne tenant pas compte des micro-colonies qui se développent parfois ni de la présence de moins de 4 colonies d'aspect

normal.

On a obtenu les résultats suivants avec des composés de l'invention:

A. - Vis-à-vis de souches de Staphylococcus aureus résistant à la triméthoprime

Les composés sous forme basique, des Exemples 1 et 4 ont été étudiés comparativement à la triméthoprime (TMP) à l'égard de 4 souches provenant de malades hospitalisés.

TABLEAU I

| Souches (No.) | CMI en µg/ml des composés : | | |
|---|---|---|---|
| | TMP | Ex. 1 | Ex. 4 |
| 40314 | 64 | 0,12 | < 0,06 |
| 23543 | 16 | 2 | 2 |
| 57760 | > 64 | 1 | 2 |
| 33274 | > 64 | < 0,06 | 0,25 |

On a également testé les composés de l'invention sous forme de sel en l'occurence sous forme d'iséthionate.

A cet effet, on a solubilisé les composés à étudier sous forme basique dans un mélange comprenant 9 ml d'eau distillée, 1 ml de diméthylformamide et quelques gouttes d'acide iséthionique.

Les résultats ci-dessous ont été obtenus comparativement à l'iséthionate de triméthoprime et à la diamino-2,4 méthoxy-3 cyclopentyloxy-4 benzyl)-5 pyrimidine (Eur. J. Med. Chem. - Chim. Ther. 1982, 17, No. 6 pp. 497-504) sous forme d'iséthionate ci après dénommé Composé X.

TABLEAU II

| Souches (No.) | CMI en µg/ml des composés : | | | | |
|---|---|---|---|---|---|
| | Iséthionate de TMP | Ex. 18 | Ex. 21 | Ex. 24 | Composé X |
| 82 | 16 | 2 | 1 | 0,5 | 32 |
| 87 | 8 | 2 | 1 | 1 | 8 |
| 101 | 16 | – | 2 | 0,5 | 32 |
| 102 | 16 | 4 | 1 | 0,5 | 32 |
| 107 | 64 | 4 | 4 | – | – |
| 109 | 8 | – | 0,5 | – | – |

L'ensemble des résultats montre la très nette supériorité des composés de l'invention sur la triméthoprime ou ses sels hydrosolubles à l'égard de souches pathogènes resistantes.

B. - Vis-à-vis de souches sensibles à la triméthoprime

1) On a enregistré les résultats suivants avec les composés, sous forme basique, des Exemples 1 et 4 comparativement à la triméthoprime.

TABLEAU III

a) Bactéries Gram +

| Souches (No) | CMI en µg/ml des composés : TMP | Ex. 1 | Ex. 4 |
|---|---|---|---|
| Staphylococcus aureus ATCC 25923 | 1 | 0,25 | 0,125 |
| Staphylococcus aureus ATCC 6538 P | 0,125 | < 0,06 | < 0,06 |
| Staphylococcus aureus 18839 | 0,5 | 0,125 | 0,125 |

b) Bactéries Gram -

| Souches (No) | TMP | Ex. 1 | Ex. 4 |
|---|---|---|---|
| Escherichia coli ATCC 25922 | 4 | > 32 | > 32 |
| Escherichia coli 39949 | 0,5 | > 32 | > 32 |
| Serratia marcescens 56644 | 4 | > 32 | > 32 |

2) On a enregistré les résultats suivants avec des composés de l'invention sous forme de sel en l'occurence l'iséthionate comparativement à l'iséthionate de triméthoprime et au Composé X.

TABLEAU IV

a) Bactéries Gram +

| Souches (No.) | CMI en µg/ml des composés | | | |
|---|---|---|---|---|
| | Iséthionate de TMP | Ex. 21 | Ex. 24 | Composé X |
| Staphylococcus aureus ATCC 25923 | 0,25 | 0,5 | 0,125 | 1 |
| Staphylococcus aureus ATCC 6338 | 0,06 | 0,06 | 0,06 | 0,25 |
| Staphylococcus aureus 18839 | 0,125 | 0,125 | 0,06 | 0,5 |
| Listeria monocytogenes 5921 | 0,125 | 0,06 | 0,06 | 0,125 |
| Listeria monocytogenes 6777 | 0,125 | 0,06 | 0,06 | 0,125 |
| Listeria monocytogenes 378 | 0,125 | 0,06 | 0,06 | 0,25 |
| Listeria monocytogenes 485 | 0,125 | 0,06 | 0,06 | 0,25 |

b) Bactéries Gram -

| | | | | |
|---|---|---|---|---|
| Escherichia coli ATCC 25922 | 4 | 32 | > 64 | > 64 |
| Escherichia coli 33430 | 8 | 16 | > 64 | > 64 |
| Serratia marcescens 56644 | 2 | 16 | > 64 | 32 |

L'activité des composés de l'invention ainsi que de dérivés de benzyl-pyrimidine à l'égard de 50 souches de Staphylococcus aureus isolées de prélèvements provenant de malades hospitalisés a également été testée.

On a exprimé ci-dessous les résultats sous forme d' "activité" A, celle-ci étant définie comme le logarithme décimal de l'inverse de la CMI exprimé en µmole/ml :

$$A = \log \frac{M}{CMI\ (\mu g/ml)}$$

M représentant la masse molaire de la molécule.

Une différence de CMI d'une dilution correspond à une différence d'activité de 0,3.

L'activité des composés rapportée dans les Tableaux ci-dessous a été calculée à partir de la "moyenne" des CMI de l'ensemble des souches testées. Les CMI étant toujours très voisines d'une souche à l'autre pour un même composé, on peut admettre que cette "moyenne" représente une grandeur significative.

1) On a obtenu les résultats suivants avec des composés de l'invention, sous forme basique comparativement à la triméthoprime.

TABLEAU V

| | TMP | Composés : Ex. 1 | Ex. 4 |
|---|---|---|---|
| Moyenne des activités | 2,34 | 2,82 | 2,85 |
| Moyenne des activités rapportées à celle de la TMP prise comme unité | 1 | 1,21 | 1,22 |

2) On a également obtenu les résultats suivants avec des composés de l'invention sous forme de lactobionate comparativement au lactobionate de triméthoprime.

TABLEAU 6

| | Lactobionate de TMP | Composés : Ex. 8 | Ex. 12 | Ex. 9 | Ex. 13 | Ex. 14 | Ex. 11 |
|---|---|---|---|---|---|---|---|
| Moyenne des activités | 2,80 | 3,44 | 3,42 | 3,30 | 3,41 | 3,73 | 3,29 |
| Moyenne des activités rapportées à celle du lactobionate de TMP prise comme unité | 1 | 1,23 | 1,22 | 1,18 | 1,22 | 1,33 | 1,18 |

3) On a obtenu les résultats suivants avec des composés de l'invention sous forme d'iséthionate comparativement à l'iséthionate de triméthoprime et au Composé X.

TABLEAU VII

| | Iséthionate de TMP | Composés : Ex. 21 | Ex. 24 | Composé X |
|---|---|---|---|---|
| Moyenne des activités rapportées à celle de l'iséthionate de TMP prise comme unité | 1 | 0,25 | 0,25 | 2 |

L'ensemble de ces résultats montre la très grande sensibilité et spécificité des composés de l'invention à l'égard de bactéries Gram + en particulier à l'égard de souches de Staphylococcus aureus.

II. Evaluation de l'activité bactériostatique par diffusion en milieu gélosé

On utilise à cet effet le milieu gélosé de MUELLER-HINTON que l'on ensemence par inondation à partir d'une suspension bactérienne titrant 5 x $10^6$ à 1 x $10^7$ unités formant colonies par ml. On imprègne les disques (disque de 6 mm de diamètre de SCHLEICHER et SCHULL) avec 20 µl d'une solution de chaque composé à étudier de façon à obtenir une charge de 5 µg par disque. A titre de contrôle lors de chaque manipulation, on charge également un disque avec 5 µg de triméthoprime. On néglige également dans ce test les microcolonies

qui peuvent apparaître à l'intérieur des zones d'inhibition.

On a obtenu les résultats suivants exprimés en mm d'inhibition, avec des composés de l'invention sous forme basique comparativement à la triméthoprime. Ces résultats ont été enregistrés vis-à-vis de souches de Staphylococcus aureus résistant à la triméthoprime.

TABLEAU VIII

| Souches (No.) | Diamètre d'inhibition (mm) dus aux composés | | |
|---|---|---|---|
| | TMP | Ex. 1 | Ex. 4 |
| 40314 | 0 | 32 | 35 |
| 23543 | 0 | 19 | 19 |
| 57760 | 0 | 24 | 20 |
| 33274 | 0 | 32 | 29 |

Ces résultats confirment l'activité antibactérienne des composés de l'invention vis-à-vis de souches résistant à la triméthoprime.

III. Evaluation de l'activité des composés de l'invention en association avec des dérivés de sulfamide

L'action du composé de l'Exemple 21 a été étudiée comparativement à l'iséthionate de triméthoprime vis-à-vis de 73 souches de bacilles à Gram -. Des résultats trouvés, on a pu conclure que 33 souches étaient résistantes au sel de triméthoprime et 65 souches étaient résistantes au composé de l'Exemple 21.

Par contre, si on associe chacun de ces composés au sulfaméthoxazole, le spectre d'activité de l'association composé de l'Exemple 21/sulfaméthoxazole est similaire à celui de l'association de triméthoprime/ sulfaméthoxazole.

Par conséquent, le composé de l'Exemple 21 potentialise aussi bien le sulfaméthoxazole que ne le fait le sel de triméthoprime.

IV. Toxicité

La toxicité aiguë des composés de l'invention a été déterminée sur des lots de 5 souris par voie intraveineuse. On a ainsi trouvé que la $DL_{50}$du composé de l'Exemple 9, selon cette voie d'administration, est de 157,4 mg/kg.

Les compositions thérapeutiques de l'invention peuvent être présentées sous toutes formes convenant à l'administration en thérapie humaine ou vétérinaire.

Pour ce qui concerne l'unité d'administration, celle-ci peut prendre la forme, par exemple d'un comprimé dragéifié ou non, d'une capsule, d'une gélule, d'une poudre, d'un granulé, d'une suspension ou d'un sirop pour l'administration orale, d'un suppositoire pour l'administration rectale ou d'une solution stérile ou suspension pour l'administration parentérale.

La quantité totale de dérivé de benzyl-pyrimidine selon l'invention peut varier entre 20 et 80 mg par unité d'administration le dérivé de benzyl-pyrimidine pouvant être seul ou en mélange avec d'autres principes actifs tels que des dérivés de sulfamide.

Suivant la voie d'administration choisie, les compositions pharmaceutiques ou vétérinaires de l'invention seront préparées en associant au moins un des composés de formule I ou un sel biologiquement acceptable de ce composé avec un véhicule ou un excipient approprié, ce dernier pouvant être constitué par exemple, d'au moins un ingrédient sélectionné parmi les substances suivantes : lactose, amidons, talc, stéarate de magnésium, polyvinylpyrrolidone, silice colloïdale, cellulose microcristalline, p-hydroxybenzoate de méthyle ou de propyle, glycérol, eau distillée, métabisulfite de sodium, propylèneglycol, éthanol, alcool benzylique, beurre de cacao, agents édulcorants et similaires.

Les Exemples non limitatifs suivants illustrent l'invention :

PREPARATIONS

a) β-Anilino α-(méthoxy-3 éthoxy-4 benzyl)-acrylonitrile

On ajoute 18 g (0,1 mole) de méthoxy-3 éthoxy-4 benzaldéhyde à 50 ml de diméthylsulfoxyde contenant 16 g (0.11 mole) de β-anilinopropionitrile.On refroidit le mélange à 15° C et on ajoute goutte à goutte une solution obtenue en dissolvant 4 g de potassium dans 90 ml de tertiobutanol. On chauffe durant 1 h à 50° C puis on élimine l'alcool sous vide. On ajoute alors 25 ml d'un mélange éthanol/eau 50/50 puis de l'eau jusqu'à apparition d'un trouble persistant. Après 6 à 8 h au froid, on sépare le solide formé et on le lave avec le minimum d'éthanol/eau 50/50 glacé.

On obtient ainsi le β-anilino (méthoxy-3 éthoxy-4 benzyl)-acrylonitrile.
P.F. : 130 - 135°C (toluène)

De la même manière que précédemment au départ des produits appropriés, on a préparé les composés suivants :

Composés

β-Anilino α-(diéthoxy-3,4 benzyl)-acrylonitrile
P.F. : 116°C
β-Anilino α-(méthoxy-3 isopropyl-4 benzyl)-acrylonitrile
P.F. : 150 - 151°C
β-Anilino α-(méthoxy-3 cyclopentyloxy-4 benzyl)-acrylonitrile
Huile jaune

b) Diamino-2,4 (méthoxy-3 éthoxy-4 benzyl)-5 pyrimidine

On dissout 2,8 g (0,12 at.gr.) de sodium dans 100 ml d'éthanol puis on ajoute 9,6 g (0,1 mole) de chlorhydrate de guanidine. Après 15 min. d'agitation, on élimine, par filtration, le chlorure de sodium formé et on ajoute au filtrat 15,4 g (0,05 mole) de β-anilino α-(méthoxy-3 éthoxy-4 benzyl)-acrylonitrile. On porte le mélange au reflux pendant 20 h puis on refroidit, ce qui provoque la précipitation du composé désiré que l'on filtre, lave à l'eau, à l'éthanol et à l'éther. Après recristallisation dans l'éthanol, on obtient la diamino-2,4 (méthoxy-3 éthoxy-4 benzyl)-5 pyrimidine. P.F. : 192-194°C.

De la même manière que précédemment, au départ des produits appropriés, on a préparé les composés suivants :

Composés

Diamino-2,4 (diéthoxy-3,4 benzyl)-5 pyrimidine
P.F. : 188°C
Diamino-2,4 (méthoxy-3 isopropyl-4 benzyl)-5 pyrimidine
P.F. : 133°C
Diamino-2,4 (méthoxy-3 cyclopentyloxy-4 benzyl)-5 pyrimidine
P.F. : 165°C

EXEMPLE 1

Préparation de la diamino-2,4 (iodo-2 éthoxy-4 méthoxy-5 benzyl)-5 pyrimidine

On dissout 2,74 g (0,01 mole) de diamino-2,4 (méthoxy-3 éthoxy-4 benzyl)-5 pyrimidine dans 40 ml d'acide acétique.

On ajoute alors goutte à goutte une solution de 1,8 g (0,11 mole ) de monochlorure d'iode dans 10 ml d'acide acétique puis 100 ml d'eau. On chauffe lentement jusqu'à ébullition puis on maintient la température pendant 30 min.. Après refroidissement, on filtre le précipité blanc. On le dissout dans 100 ml d'eau bouillante et on ajoute une solution d'hydroxyde de sodium à 10% jusqu'à pH basique. On abandonne la suspension au froid pendant 6 à 8 h. On filtre le précipité formé, lave à l'eau puis à l'éthanol.

Après recristallisation dans l'éthanol, on obtient la diamino-2,4 (iodo-2 éthoxy-4 méthoxy-5 benzyl)-5 pyrimidine.
P.F. : 218-220°C
Spectre R.M.N (resonance magnétique nucléaire) ($DMSOD_6$)
t à 1,30 (3H), s à 3,58 (2H), s à 3,69 (3H), q à 4,00 (2H), s à 5,24 (2H), s à 5,64 (2H), s à 6,77 (1H), s à 7,25 (1H), s à 7,30 (1H).

De la même manière que précédemment, au départ des produits appropriés, on a préparé les composés suivants :

Composés

Diamino-2,4 (iodo-2 diéthoxy-4,5 benzyl)-5 pyrimidine (Exemple 2)
P.F. : 214 - 215°C
Spectre R.M.N. ($DMSOD_6$)
m à 1,2-1,4 (6H), s à 3,60 (2H), m à 3,88-4,18 (4H), s à 5,80 (2H), s à 6,29 (2H), s à 6,92 (1H), s à 7,19 (1H), s à 7,40 (1H).
Diamino-2,4 (iodo-2 isopropyl-4 méthoxy-5 benzyl)-5 pyrimidine (Exemple 3)
P.F. : 238 - 240°C
Spectre R.M.N. ($DMSOD_6$)
d à 1,14 (6H), m à 3,17 (1H), s à 3,57 (2H), s à 3,70 (3H), s à 5,67 (2H), s à 6,19 (2H), s à 6,82 (1H), s à 7,06 (1H), s à 7,52 (1H)
Diamino-2,4 (iodo-2 cyclopentyloxy-4 méthoxy-5 benzyl)-5 pyrimidine (Exemple 4)
P.F. : 208 - 210°C
Spectre R.M.N. ($DMSOD_6$)
m à 1,4-1,8 (8H), s à 3,60 (2H), s à 3,70 (3H), m à 4,76 (1H), s à 6,69 (2H) et 7,16 (2H), s à 6,82 (1H), s à 6,88 (1H), s à 7,29 (1H)

EXEMPLE 5

Préparation de la diamino-2,4 (bromo-2 diéthoxy-4,5 benzyl)-5 pyrimidine

On dissout 2,88 g (0,01 mole) de diamino-2,4 (diéthoxy-4,5 benzyl)-5 pyrimidine dans 40 ml d'acide acétique et on y ajoute goutte à goutte une solution de 0,6 ml (0,012 mole) de brome dans 20 ml d'acide acétique. Après 2 h d'agitation à température ambiante, on filtre le précipité et on le lave à l'acide acétique puis à l'éthanol. On le dissout dans 200 ml d'eau bouillante à laquelle on ajoute une solution d'hydroxyde de sodium à 10% jusqu'à pH basique. On abandonne la suspension durant 6 à 8 heures au froid puis on filtre le précipité. On le lave à l'eau puis à l'éthanol.

On obtient ainsi la diamino-2,4 (bromo-2 diéthoxy-4,5 benzyl)-5 pyrimidine après recristallisation dans l'éthanol.

P.F. : 225°C

Spectre R.M.N. ($DMSOD_6$)

m à 1,2-1,4 (6H), s à 3,58 (2H), m à 3,83-4,14 (4H), s à 5,72 (2H), s à 6,20 (2H), s à 6,86 (1H), s à 7,15 (2H)

De la même manière que précédemment, au départ des produits appropriés, on a préparé les composés suivants :

Composés

Diamino-2,4 (bromo-2 isopropyl-4 méthoxy-5 benzyl)-5 pyrimidine (Exemple 6)

P.F. : 225 - 226°C

Spectre R.M.N. ($DMSOD_6$)

d à 1,18 (6H), m à 3,19 (1H), s à 3,64 (2H), s à 3,72 (3H), s à 5,68 (2H), s à 6,17 (2H), s à 6,87 (1H), s à 7,17 (1H), s à 7,32 (1H)

Diamino-2,4 (bromo-2 cyclopentyloxy-4 méthoxy-5 benzyl)-5 pyrimidine (Exemple 7)

P.F. : 188 - 189°C

Spectre R.M.N. ($DMSOD_6$)

m à 1,5-1,8 (8H), s à 3,58 (2H), m à 4,80 (1H), s à 5,70 (2H), s à 6,18 (2H), s à 6,87 (1H), s à 7,10 (1H), s à 7,15 (1H)

EXEMPLE 8

Préparation du lactobionate de diamino-2,4 (iodo-2 éthoxy-4 méthoxy-5 benzyl)-5 pyrimidine

On dissout 0,2 g ($5.10^{-4}$ mole) de diamino-2,4 (iodo-2 éthoxy-4 méthoxy-5 benzyl)-5 pyrimidine et 0,1790 g d'acide lactobionique dans 5 ml d'eau distillée à 50°C. On ajoute alors 2 ml d'éthanol. On filtre la solution limpide et on évapore sous vide. On obtient 0,37 g de lactobionate de diamino-2,4 (iodo-2 éthoxy-4 méthoxy-5 benzyl)-5 pyrimidine sous forme de poudre blanche.

P.F. : 130 - 135°C

De la même manière que précédemment, au départ des produits appropriés, on a préparé les composés suivants :

Composés

Lactobionate de diamino-2,4 (iodo-2 diéthoxy-4,5 benzyl)-5 pyrimidine (Exemple 9)

P.F. : 95 - 110°C

Lactobionate de diamino-2,4 (iodo-2 isopropyl-4 méthoxy-5 benzyl)-5 pyrimidine (Exemple 10)

P.F. : 120 - 200°C (décomposition)

Lactobionate de diamino-2,4 (iodo-2 cyclopentyloxy-4 méthoxy-5 benzyl)-5 pyrimidine (Exemple 11)

P.F. : 176-180°C

Lactobionate de diamino-2,4 (bromo-2 diéthoxy-4,5 benzyl)-5 pyrimidine (Exemple 12)

P.F. : 80 - 110°C (décomposition)

Lactobionate de diamino-2,4 (bromo-2 isopropyl-4 méthoxy-5 benzyl)-5 pyrimidine (Exemple 13)

P.F. : 204°C

Lactobionate de diamino-2,4 (bromo-2 cyclopentyloxy-4 méthoxy-5 benzyl)-5 pyrimidine (Exemple 14)

P.F. : 170-180°C

Lactobionate de diamino-2,4 (bromo-2 cyclohexyloxy-4 méthoxy-5 benzyl)-5 pyrimidine (Exemple 23)

EXEMPLE 15

Iséthionate de diamino-2,4 (iodo-2 diéthoxy-4,5 benzyl)-5 pyrimidine

a) Acide iséthionique

Cet acide est obtenu à partir de son sel de sodium par passage d'une solution aqueuse de celui-ci sur une colonne chromatographique garnie de résine échangeuse de cations de type acide. On obtient ainsi une solution d'acide iséthionique 0,60 N.

b) Iséthionate de diamino-2,4 (iodo-2 diéthoxy-4,5 benzyl)-5 pyrimidine

Sous agitation, on ajoute 0,72 g ($17 \times 10^{-4}$ mole) de diamino-2,4 (iodo-2 diéthoxy-4,5 benzyl)-5 pyrimidine à 3 ml d'acide iséthionique 0,60 N. On concentre la solution sous vide jusqu'à un volume de 1 ml environ. On

ajoute quelques gouttes d'éthanol puis quelques gouttes d'éther éthylique jusqu'à l'apparition d'un précipité blanc.

De cette manière on obtient 0,5 g d'iséthionate de diamino-2,4 (iodo-2 diéthoxy-4,5 benzyl)-5 pyrimidine.
P.F. : 206°C

De la même manière que précédemment, au départ des produits appropriés, on a préparé les composés suivants :

Composés
Iséthionate de diamino-2,4 (iodo-2 éthoxy-4 méthoxy-5 benzyl)-5 pyrimidine (Exemple 16)
Iséthionate de diamino-2,4 (iodo-2 isopropyl-4 méthoxy-5 benzyl)-5 pyrimidine (Exemple 17)
Iséthionate de diamino-2,4 (iodo-2 cyclopentyloxy-4 méthoxy-5 benzyl)-5 pyrimidine (Exemple 18)
Iséthionate de diamino-2,4 (bromo-2 diéthoxy-4,5 benzyl)-5 pyrimidine (Exemple 19)
Iséthionate de diamino-2,4 (bromo-2 isopropyl-4 méthoxy-5 benzyl)-5 pyrimidine (Exemple 20)
Iséthionate de diamino-2,4 (bromo-2 cyclopentyloxy-4 méthoxy-5 benzyl)-5 pyrimidine (Exemple 21)
Iséthionate de diamino-2,4 (bromo-2 cyclohexyloxy-4 méthoxy-5 benzyl)-5 pyrimidine (Exemple 24)

EXEMPLE 22

Préparation de diamino-2,4 (bromo-2 cyclohexyloxy-4 méthoxy-5 benzyl)-5 pyrimidine

a) Méthoxy-3 cyclohexyloxy-4 benzaldéhyde

Dans un ballon d'un litre muni d'un puissant agitateur mécanique, d'un thermomètre et d'un calotte chauffante, on place 25 g (0,16 mole) de vanilline, 125 ml de diméthylformamide anhydre, 21 ml (0,17 mole) de bromocyclohexane, 27,5 g (0,2 mole) de carbonate de potassium et on agite fortement le mélange au reflux du diméthylformamide pendant 36 heures. On ajoute ensuite 40 ml de bromocyclohexane et 20 g de carbonate de potassium et on poursuit l'agitation pendant 8 à 10 heures. Après refroidissement, on évapore le diméthylformamide à sec et on reprend le résidu dans du toluène et de l'eau. On extrait la phase aqueuse deux fois avec du toluène puis une fois avec du chloroforme. On réunit les phases organiques on les lave avec une solution d'hydroxyde de sodium puis avec de l'eau. On sèche sur sulfate de sodium puis on évapore à sec.

De cette manière, on recueille le méthoxy-3 cyclohexyloxy-4 benzaldéhyde sous forme d'une huile que l'on distille.

Rendement : 18%.
C.C.M. (chromatographie sur couche mince) ($CHCl_3$)
Rf = 0,58
Spectre R.M.N. ($CDCl_3$)
m à 1,7 ppm (10H), s à 3,9 ppm (3H), m à 4,4 ppm (1H), m à 7,2 ppm (3H),
s à 10 ppm (1H)

b) α-Anilinométhyl β-(méthoxy-3 cyclohexyloxy-4 phényl)-acrylonitrile et β-anilino α-(méthoxy-3 cyclohexyloxy-4 benzyl)-acrylonitrile

Dans un ballon de 250 ml muni d'un agitateur mécanique, d'une calotte chauffante, d'un réfrigérant et d'une arrivée d'azote, on place 20 g (0,085 mole) de méthoxy-3 cyclohexyloxy-4 benzaldéhyde et 13,2 g (0,09 mole) de β-anilinopropionitrile dans 80 ml de méthanol anhydre.

Dans un erlenmeyer muni d'une garde à chlorure de calcium, on introduit 40 ml de méthanol anhydre et on y ajoute, par petites portions, 2,1 g (0,09 mole) de sodium. Quand tout le sodium est consommé, on ajoute la solution obtenue, goutte à goutte par l'intermédiaire d'une ampoule à brome au mélange aldéhyde/β-anilinopropionitrile. Après l'ajout, on chauffe au reflux pendant 24 heures et on suit la réaction par chromatographie sur couche mince.

On évapore à sec le mélange réactionnel et on reprend le résidu dans du toluène et de l'eau. On extrait la phase aqueuse deux fois avec du toluène puis une fois avec du chloroforme. On réunit les phases organiques, on les lave à l'eau puis avec une solution d'acide chlorhydrique dilué et finalement avec de l'eau. On sèche et on évapore à sec.

On obtient ainsi 22 g d'une huile (rendement : 72%) constituée d'un mélange d'α-anilinométhyl β-(méthoxy-3 cyclohexyloxy-4 phényl)-acrylonitrile/β-anilino α-(méthoxy-3 cyclohexyloxy-4 benzyl)-acrylonitrile que l'on peut utiliser tel quel pour l'étape suivante. Si nécessaire, ce mélange peut être séparé par chromatographie sur colonne (éluant : éther éthylique/hexane 15/5) pour obtenir :

α-Anilinométhyl β-(méthoxy-3 cyclohexyloxy-4 phényl)-acrylonitrile (composé α)
P.F. : 52°C (diméthylformamide)
β-Anilino α-(méthoxy-3 cyclohexyloxy-4 benzyl)-acrylonitrile (composé β)
P.F. : 98°C (éther éthylique/hexane)
C.C.M. (éther éthylique/hexane 15/5) :

Rf
Composé α 0,58
Composé β 0,75
Benzaldéhyde de départ 0,80

c) Diamino-2,4 (méthoxy-3 cyclohexyloxy-4 benzyl)-5 pyrimidine

Dans un erlenmeyer de 250 ml muni d'un agitateur magnétique chauffant, d'un tube d'introduction d'azote et d'un réfrigérant, on place 5,6 g (0,24 at.gr.) de sodium dans 180 ml de méthanol anhydre et on y ajoute 17,4 g (0,18 mole) de chlorhydrate de guanidine. Après 15 min. d'agitation, on ajoute 22 g du mélange de composés α et β obtenu précédemment. On chauffe, au reflux pendant 30 min., la solution obtenue puis on la place au réfrigérateur pendant 8 à 10 heures. On filtre le précipité formé, on le lave à l'eau plusieurs fois avec de l'éthanol froid puis avec de l'éther éthylique et on le sèche dans un dessicateur.

De cette manière, on recueille 7 g de diamino-2,4 (méthoxy-3 cyclohexyloxy-4 benzyl)-5 pyrimidine.

Rendement : 35%

P.F. : 170°C

C.C.M. (chloroforme/méthanol) :

Rf = 0,6

Spectre R.M.N. ($DMSOD_6$)

m à 1,5 ppm (10H), s à 3,6 ppm (2H), s à 3,8 ppm (3H), m à 4,2 ppm (1H), s à 5,7 ppm (2H) ($NH_2$), s à 6,1 ppm (2H) ($NH_2$), m à 7,0 ppm (3H), s à 7,5 ppm (1H)

d) Diamino-2,4 (bromo-2 cyclohexyloxy-4 méthoxy-5 benzyl)-5 pyrimidine

Dans un erlenmeyer de 100 ml muni d'une ampoule à brome, d'un réfrigérant, d'un agitateur mécanique chauffant, on place 2,5 g (0,0076 mole) de diamino-2,4 (méthoxy-3 cyclohexyloxy-4 benzyl)-5 pyrimidine dans 30 ml d'acide acétique et on chauffe le mélange obtenu pour permettre la dissolution du dérivé de pyrimidine. Après solubilisation, on ramène la solution à température ambiante et on y ajoute, par l'intermédiaire de l'ampoule à brome, 0,5 ml (0,0021 mole) de brome dilué dans 15 ml d'acide acétique. On maintient alors l'agitation pendant 6 heures à température ambiante. On évapore la solution à sec, on reprend le résidu avec un minimum d'eau chaude et on ramène le mélange formé à température ambiante. On rend le milieu basique par ajout d'hydroxyde de sodium et on le place au réfrigérateur pendant 8 à 10 heures. On filtre le précipité formé, lave à l'eau puis avec de l'éthanol et de l'éther éthylique. Après sèchage, on recueille 1,4 g de diamino-2,4 (bromo-2 cyclohexyloxy-4 méthoxy-5 benzyl)-5 pyrimidine.

Rendement : 45%

P.F. : 232°C

C.C.M. (chloroforme/éthanol 18/2) :

Rf = 0,37

Spectre R.M.N. ($DMSOD_6$)

m à 1,5 ppm (10H), s à 3,6 ppm (2H), s à 3,8 ppm (3H), m à 4,2 ppm (1H), s à 5,7 ppm (2H) ($NH_2$), s à 6,2 ppm (2H) ($NH_2$), s à 6,9 ppm (1H), s à 7,2 ppm (2H).

EXEMPLE 25

On a préparé une composition destinée à l'administration parentérale ayant la formule centésimale suivante :

Composé de l'invention 4 g

Sulfadiméthoxine sodique 20 g

Triéthanolamine 85% 2 ml

p-Hydroxybenzoate de méthyle 0,1 g

Diméthylacétamide 30 ml

Eau 13 ml

Glycerolformal q.s.p. 100 ml

a) on dissout sous agitation dans 13 l d'eau purifiée, 2,4 kg d'hydroxyde de sodium, 0,1 kg de p-hydroxybenzoate de méthyle et 2 l de triéthanolamine 85%. Quand la dissolution est complète, on porte la solution à 50°C et on ajoute 18,67 kg de sulfadiméthoxine acide et on agite.

b) Dans la cuve principale de fabrication on verse 30 l de diméthylacétamide et 15 l de glycérolformal. On chauffe à 65°C et on dissout le composé de l'invention. Sous agitation on ajoute la solution de sulfadiméthoxine sodique obtenue au paragraphe a) précédent et on complète par la quantité nécessaire de glycérolformal pour atteindre 100 l. On agite puis on filtre. On répartit en flacons puis on stérilise.

EXEMPLE 26

Suivant des techniques pharmaceutiques connues, on a préparé une composition pour l'administration orale comprenant 200 mg de composé de l'invention avec comme excipient le glycolate d'amidon sodique, l'amidon de maïs et le stéarate de magnésium.

**Revendications**

1. Dérivés de benzyl-pyrimidine de formule générale :

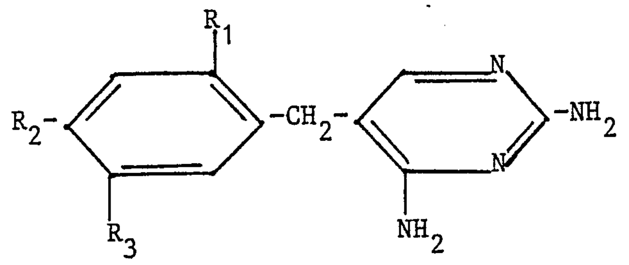

ainsi que leurs sels biologiquement acceptables, dans laquelle $R_1$ représente un atome de brome ou d'iode, $R_2$ représente un groupement isopropyle, éthoxy ou cycloalkoxy ayant de 3 à 6 atomes de carbone et $R_3$ représente un radical alkoxy ayant de 1 à 3 atomes de carbone, à condition que lorsque $R_2$ représente éthoxy et $R_3$ représente méthoxy, $R_1$ représente iode.

2. Diamino-2,4 (iodo-2 éthoxy-4 méthoxy-5 benzyl)-5 pyrimidine et ses sels biologiquement acceptables.

3. Diamino-2,4 (bromo-2 cyclopentyloxy-4 méthoxy-5 benzyl)-5 pyrimidine et ses sels biologiquement acceptables.

4. Diamino-2,4 (iodo-2 cyclopentyloxy-4 methoxy-5 benzyl)-5 pyrimidine et ses sels biologiquement acceptables.

5. Diamino-2,4 (bromo-2 cyclohexyloxy-4 méthoxy-5 benzyl)-5 pyrimidine et ses sels biologiquement acceptables.

6. Dérivés de benzyl-pyrimidine selon l'un quelconque des revendications 1 à 5 caractérisés en ce que le sel biologiquement acceptable est le lactobionate.

7. Dérivés de benzyl-pyrimidine selon l'une quelconque des revendications 1 à 5, caractérisés en ce que le sel biologiquement acceptable est l'iséthionate.

8. Procédé de préparation de dérivés de benzyl-pyrimidine selon l'une quelconque des revendications 1 à 7, caractérisé en ce que l'on halogène en présence d'acide acétique un dérivé de benzyl-pyrimidine de formule générale :

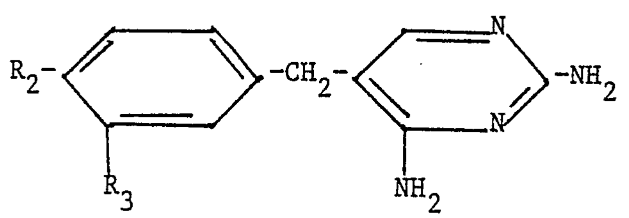

dans laquelle $R_2$ et $R_3$ ont la même signification que dans la revendication 1, avec le brome ou le monochlorure d'iode pour obtenir le composé désiré sous forme basique que l'on fait réagir, si on le désire, avec un acide organique ou inorganique approprié pour obtenir un sel biologiquement acceptable de ce composé.

9. Composition pharmaceutique ou vétérinaire contenant, comme principe actif, au moins un dérivé de benzyl-pyrimidine selon l'une quelconque des revendications 1 à 7, en association avec un véhicule pharmaceutique ou un excipient approprié.

10. Composition pharmaceutique ou vétérinaire contenant, comme principe actif, au moins un dérivé de benzyl-pyrimidine selon l'une quelconque des revendications 1 à 7, en association avec un dérivé de sulfamide ainsi qu'avec un véhicule pharmaceutique ou un excipient approprié.

11. Composition pharmaceutique ou vétérinaire ayant des propriétés antibactériennes spécifiques des bactéries Gram +, antiparasitaires et antipaludéennes contenant comme principe actif au moins un dérivé de benzyl pyrimidine selon l'une quelconque des revendications 1 à 7, en association avec un véhicule pharmaceutique ou un excipient approprié.

Office européen des brevets

# RAPPORT DE RECHERCHE EUROPEENNE

Numéro de la demande

EP 87 87 0032

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EUR. J. MED. CHEM. - CHIM. THER., vol. 17, no. 6, 1982, pages 497-504, Paris, FR; M. CALAS et al.: "Synthèse d'analogues nouveaux de la triméthoprime"<br>* Page 497; page 498, tableau I, no. 26; pages 499-502 * | 1,5,8-10 | C 07 D 239/49<br>A 61 K 31/505 |
| A | DE-B-1 204 678 (WELLCOME)<br>* Colonne 1; colonne 4, exemple 2; colonnes 5,6 * | 1,8 | |
| A | FR-M- 5 411 (HOFFMANN-LA ROCHE)<br>* Pages 1,8 * | 1,8 | |
| A | FR-A-1 292 920 (WELLCOME)<br>* Page 1; page 3, exemples 8,9,13 * | 1,8 | DOMAINES TECHNIQUES RECHERCHES (Int. Cl.4)<br>C 07 D 239/00 |

Le présent rapport de recherche a été établi pour toutes les revendications

| Lieu de la recherche | Date d'achèvement de la recherche | Examinateur |
|---|---|---|
| LA HAYE | 15-06-1987 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82